# EUROPÄISCHE PATENTSCHRIFT

(11) **EP 3 197 987 B1**
(45) Veröffentlichungstag und Bekanntmachung des Hinweises auf die Patenterteilung: **23.01.2019**
(21) Anmeldenummer: 15767522.4
(22) Anmeldetag: 25.09.2015
(51) Int. Cl.: C11B 9/00, C07D 309/10

(54) **VERWENDUNG VON ISOMERENREINEM ODER HOCH ISOMERENANGEREICHERTEM CIS- ODER TRANS-(2-ISOBUTYL-4-METHYL-TETRAHYDROPYRAN-4-YL)ACETAT**
USE OF ISOMERICALLY PURE OR HIGHLY ISOMERICALLY ENRICHED CIS- OR TRANS- (2-ISOBUTYL-4-METHYL-TETRAHYDROPYRAN-4-YL) ACETATE
UTILISATION DE CIS- OU TRANS-(2-ISOBUTYL-4-MÉTHYL-TÉTRAHYDROPYRANE-4-YL) ACÉTATE ISOMÈRIQUE PUR OU TRÈS ENRICHI EN ISOMÈRES

(30) Priorität: 26.09.2014 EP 14186627
(43) Veröffentlichungstag der Anmeldung: 02.08.2017
(73) Patentinhaber: BASF SE, 67056 Ludwigshafen (DE)
(72) Erfinder: STORK, Timon, 68642 Bürstadt Bobstadt (DE); RÜDENAUER, Stefan, 69469 Weinheim (DE); KLOS, Margarethe, 67240 Bobenheim Roxheim (DE); PELZER, Ralf, 37699 Fürstenberg (DE); KRAUSE, Wolfgang, 68782 Brühl-Rohrhof (DE)
(86) Internationale Anmeldenummer: PCT/EP2015/072083
(87) Internationale Veröffentlichungsnummer: WO 2016/046360

(56) Entgegenhaltungen:
- EP-A2- 0 383 446

## Beschreibung

### HINTERGRUND DER ERFINDUNG

Die vorliegende Erfindung betrifft die Verwendung von isomerenreinem oder hoch isomerenangereichertem cis- oder trans-(2-Isobutyl-4-methyl-tetrahydropyran-4-yl)acetat für den Einsatz als Aromachemikalien. Die Erfindung betrifft weiterhin ein Verfahren zur Herstellung von isomerenreinem oder hoch isomerenangereichertem cis- oder trans-(2-Isobutyl-4-methyl-tetrahydropyran-4-yl)acetat und die nach diesem Verfahren erhältlichen Produkte. Die Erfindung betrifft weiterhin eine Duft- oder Aromastoffzusammensetzung, die isomerenreines oder hoch isomerenangereichertes cis- oder trans-(2-Isobutyl-4-methyl-tetrahydropyran-4-yl)acetat enthält, ein Verfahren zum Verleihen und/oder Verstärken eines Geruchs oder Geschmacks eines Produkts sowie parfümierte oder aromatisierte Produkte, die isomerenreines oder hoch isomerenangereichertes cis- oder trans-(2-lsobutyl-4-methyl-tetrahydropyran-4-yl)acetat enthalten.

### STAND DER TECHNIK

Trotz einer Vielzahl bereits vorhandener Aromachemikalien (Riech- und Geschmacksstoffe) besteht ein ständiger Bedarf an neuen Komponenten, um die Vielzahl der für die äußerst diversen Einsatzbereiche gewünschten Eigenschaften erfüllen zu können. Dazu zählen zum einen die organoleptischen Eigenschaften, d. h. die Verbindungen sollen über vorteilhafte geruchliche (olfaktorische) oder geschmackliche (gustatorische) Eigenschaften verfügen. Des Weiteren sollen Aromachemikalien aber auch zusätzliche positive Sekundäreigenschaften besitzen, wie z. B. eine effiziente Herstellungsweise, die Möglichkeit der Bereitstellung besserer sensorischer Profile durch Synergieeffekte mit anderen Riechstoffen, eine höhere Stabilität unter bestimmten Anwendungsbedingungen, eine höhere Ausgiebigkeit, ein besseres Haftungsvermögen, etc.

2-substituierte 4-Hydroxy-4-methyl-tetrahydropyrane sind bekannte wertvolle Verbindungen für den Einsatz als Aromachemikalien. So zeichnet sich beispielsweise das cis/trans-Diastereomerengemisch des 2-(2-Methylpropyl)-4-hydroxy-4-methyl-tetrahydropyrans 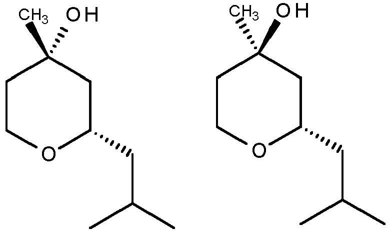 durch einen angenehmen Maiglöckchenduft aus und ist in besonderem Maße zur Verwendung als Aromachemikalie, z. B. zur Herstellung von Riechstoffkompositionen, geeignet. Wünschenswert wäre, wenn sich weitere Aromachemikalien durch einfache Derivatisierung dieser 2-substituierten 4-Hydroxy-4-methyl-tetrahydropyrane herstellen ließen, so dass neue, kostengünstige Folgeprodukte resultieren. Weiterhin wünschenswert wären auch Verbindungen, die in Kombination mit 2-substituierten 4-Hydroxy-4-methyl-tetrahydropyranen ein vorteilhaftes sensorisches Profil aufweisen.

Die EP 0383446 A2 beschreibt die Synthese sowie die olfaktorischen Eigenschaften einer Vielzahl verschiedener 2,4,4-trisubstituierter Tetrahydropyranylester. Beispiel IX beschreibt die Synthese von 2-Isobutyl-4-methyl-tetrahydropyran-4-ylacetat durch Veresterung von 2-Isobutyl-4-hydroxy-4-methyl-tetrahydropyran mit Essigsäureanhydrid in Gegenwart von Methansulfonsäure. Zwar wurde das Reaktionsprodukt einer fraktionierten Destillation unterworfen. Die erhaltenen Fraktionen wurden jedoch weder hinsichtlich der darin enthaltenen Komponenten noch bezüglich ihrer olfaktorischen Eigenschaften analysiert. Das abgebildete NMR-Spektrum (Figur 17) lässt auf ein cis/trans-Verhältnis im Bereich von 75 : 25 bis 80 : 20 schließen. Für die vereinigten Fraktionen 11 bis 17 wird ein sandelholzartiges, blumiges, rosenartiges Geruchsprofil angegeben. Dieses Dokument enthält keinen Hinweis auf die Herstellung und Eigenschaften von isomerenreinem oder hoch isomerenangereichertem cis- oder trans-(2-Isobutyl-4-methyl-tetrahydropyran-4-yl)acetat.

Der vorliegenden Erfindung liegt die Aufgabe zugrunde, neue Aromachemikalien mit vorteilhaften Eigenschaften zur Verfügung zu stellen. Diese sollen speziell über angenehme geruchliche Eigenschaften verfügen. Desweiteren sollen sie befähigt sein, in Kombination mit anderen Aromachemikalien neue vorteilhafte sensorischer Profile bereit zu stellen. Sie sollen sich zudem effektiv herstellen bzw. isolieren lassen.

Überraschenderweise wurde nun gefunden, dass diese Aufgabe durch isomerenreines oder hoch isomerenangereichertes cis- oder trans-(2-Isobutyl-4-methyl-tetrahydropyran-4-yl)acetat und dessen Verwendung als Aromachemikalie gelöst wird.

### ZUSAMMENFASSUNG DER ERFINDUNG

Gegenstand der Erfindung ist die Verwendung von isomerenreinem cis-(2-Isobutyl-4-methyl-tetrahydropyran-4-yl)acetat der Formel (I.1) oder von isomerenreinem trans-(2-Isobutyl-4-methyl-tetrahydropyran-4-yl)acetat der Formel (I.2) oder eines Isomerengemischs aus cis-(2-Isobutyl-4-methyl-tetrahydropyran-4-yl)acetat (I.1) und trans-(2-Isobutyl-4-methyl-tetrahydropyran-4-yl)acetat (I.2) 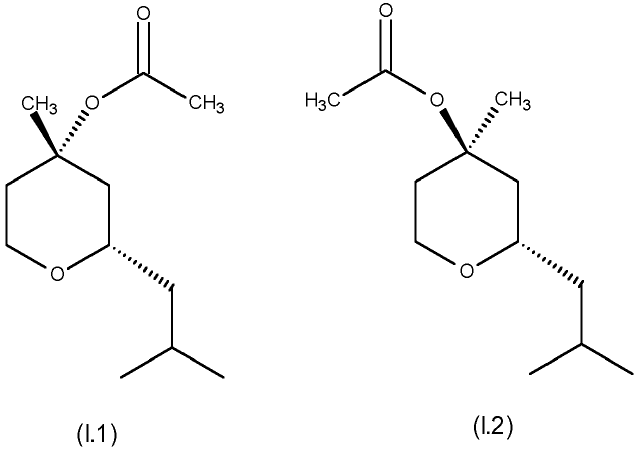 wobei bei Verwendung eines Isomerengemischs
der Gewichtsanteil von cis-(2-Isobutyl-4-methyl-tetrahydropyran-4-yl)acetat (I.1) bezogen auf das Gesamtgewicht von cis-(2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, oder
der Gewichtsanteil von trans-(2-Isobutyl-4-methyl-tetrahydropyran-4-yl)acetat (I.2) bezogen auf das Gesamtgewicht von cis-(2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt,
als Aromachemikalie.

Ein weiterer Gegenstand der Erfindung ist ein Verfahren zur Herstellung von isomerenreinem cis-(2-Isobutyl-4-methyl-tetrahydropyran-4-yl)acetat der Formel (I.1) oder von isomerenreinem trans-(2-Isobutyl-4-methyl-tetrahydropyran-4-yl)acetat der Formel (I.2) oder eines Isomerengemischs aus (I.1) und (I.2), wobei der Gewichtsanteil von (I.1) oder der Gewichtsanteil von (I.2) in dem Isomerengemisch, bezogen auf das Gesamtgewicht von (I.1) und (I.2), in einem Bereich von 90 Gew.-% bis weniger als 100 Gew.-% liegt,
bei dem man
i) ein Isomerengemisch aus cis-2-(2-Methylpropyl)-4-hydroxy-4-methyl-tetrahydropyran (II.1) und trans-2-(2-Methylpropyl)-4-hydroxy-4-methyl-tetrahydropyran (II.2) 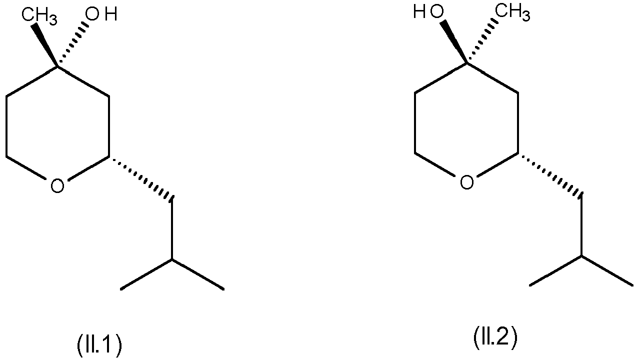 mit einer Verbindung der Formel CH₃C(=O)-X umsetzt, worin X für Cl, Br oder CH₃C(=O)O steht,
ii) gegebenenfalls das in Schritt i) erhaltene Reaktionsgemisch einer Auftrennung unter Erhalt wenigstens einer Fraktion unterzieht, die isomerenreines (I.1) oder isomerenreines (I.2) oder ein Isomerengemisch aus (I.1) und (I.2) enthält, wobei der Gewichtsanteil von (I.1) oder der Gewichtsanteil von (I.2) in dem Isomerengemisch, bezogen auf das Gesamtgewicht von (I.1) und (I.2), in einem Bereich von 90 Gew.-% bis weniger als 100 Gew.-% liegt.

Ein weiterer Gegenstand der Erfindung ist isomerenreines cis-(2-Isobutyl-4-methyl-tetrahydropyran-4-yl)acetat der Formel (I.1).

Ein weiterer Gegenstand der Erfindung ist ein Isomerengemisch aus cis-(2-Isobutyl-4-methyl-tetrahydropyran-4-yl)acetat (I.1) und trans-(2-Isobutyl-4-methyl-tetrahydropyran-4-yl)acetat (I.2) worin der Gewichtsanteil von cis-(2-Isobutyl-4-methyl-tetrahydropyran-4-yl)acetat (I.1) bezogen auf das Gesamtgewicht von cis- 2-Isobutyl-4-methyl-tetrahydropyran-4-yl)acetat (1.1) und trans-(2-Isobutyl-4-methyl-tetrahydropyran-4-yl)acetat (I.2) in einem Bereich von 90 Gew.-% bis weniger als 100 Gew.-% liegt.

Ein weiterer Gegenstand der Erfindung ist isomerenreines trans-(2-Isobutyl-4-methyl-tetrahydropyran-4-yl)acetat der Formel (I.2).

Ein weiterer Gegenstand der Erfindung ist ein Isomerengemische aus cis-(2-Isobutyl-4-methyl-tetrahydropyran-4-yl)acetat (I.1) und trans-(2-Isobutyl-4-methyl-tetrahydropyran-4-yl)acetat (I.2) worin der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt.

Ein weiterer Gegenstand der Erfindung ist eine Duft- oder Aromastoffzusammensetzung, enthaltend
a) isomerenreines cis-(2-Isobutyl-4-methyl-tetrahydropyran-4-yl)acetat der Formel (I.1) oder isomerenreines trans-(2-Isobutyl-4-methyl-tetrahydropyran-4-yl)acetat der Formel (I.2) oder ein Isomerengemisch aus cis-(2-lsobutyl-4-methyl-tetrahydropyran-4-yl)acetat (I.1) und trans-(2-Isobutyl-4-methyl-tetrahydropyran-4-yl)acetat (I.2), wobei der Gewichtsanteil von cis-(2-lsobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, oder der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt,
b) gegebenenfalls wenigstens eine weitere, von den Verbindungen (I.1) und (I.2) verschiedene Aromachemikalie, und
c) gegebenenfalls wenigstens ein Verdünnungsmittel,
mit der Maßgabe, das die Zusammensetzung wenigstens eine Komponente b) oder c) enthält.

Ein weiterer Gegenstand der Erfindung ist ein Verfahren zum Verleihen und/oder Ersetzen und/oder Verstärken eines Geruchs oder Geschmacks eines Produkts mit einer frisch-floralen Note, insbesondere einer schwertlilienartigen Note, einer Zitrusnote, insbesondere einer bergamotteartigen Note, wie z. B. der Note von Linalylacetat oder Ethyllinalylacetat, einer holzigen Note oder einer pfeffer/muskatartigen Note, bei dem das Produkt mit einer organoleptisch wirksamen Menge eines isomerenreinen cis-(2-Isobutyl-4-methyl-tetrahydropyran-4-yl)acetats der Formel (I.1) oder einem Isomerengemisch, wobei der Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, in Kontakt gebracht wird.

Ein weiterer Gegenstand der Erfindung ist ein Verfahren zum Verleihen und/oder Verstärken eines Geruchs oder Geschmacks eines Produkts mit einer jasminartigen Note, einer lederartigen Note oder einer kräftigen fruchtartigen Note, bei dem das Produkt mit einer organoleptisch wirksamen Menge eines isomerenreinen trans-(2-Isobutyl-4-methyl-tetrahydropyran-4-yl)acetats der Formel (I.2) oder einem Isomerengemisch, wobei der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, in Kontakt gebracht wird.

Ein weiterer Gegenstand der Erfindung ist ein parfümiertes oder aromatisiertes Produkt, umfassend eine organoleptisch wirksame Menge an isomerenreinem cis-(2-Isobutyl-4-methyl-tetrahydropyran-4-yl)acetat der Formel (I.1) oder an isomerenreinem trans-(2-Isobutyl-4-methyl-tetrahydropyran-4-yl)acetat der Formel (I.2) oder hoch isomerenangereichertes (I.1) oder hoch isomerenangereichertes (I.2), oder umfassend eine organoleptisch wirksame Menge einer erfindungsgemäßen Duft- oder Aromastoffzusammensetzung, wie zuvor und im Folgenden definiert.

Ein weiterer Gegenstand der Erfindung ist die Verwendung von isomerenreinem cis-(2-Isobutyl-4-methyl-tetrahydropyran-4-yl)acetat der Formel (I.1) oder eines Isomerengemischs, wobei der Gewichtsanteil von cis-(2-Isobutyl-4-methyl-tetrahydropyran-4-yl)acetat (I.1), bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, zum teilweisen oder vollständigen Ersatz von Linalylacetat in einer Linalylacetat enthaltenden Duft- oder Aromastoffzusammensetzung oder in einem mit Linalylacetat parfümierten oder aromatisierten Produkt.

### BESCHREIBUNG DER ERFINDUNG

Die Erfindung weist die folgenden Vorteile auf:
- Mit isomerenreinem oder hoch isomerenangereichertem cis- oder trans-(2-Isobutyl-4-methyl-tetrahydropyran-4-yl)acetat werden neue Verbindungen für die Verwendung als Aromachemikalie und speziell als Duftstoff bereitgestellt, die über sehr vorteilhafte olfaktorische Eigenschaften verfügen. Isomerenreines cis-(2-Isobutyl-4-methyl-tetrahydropyran-4-yl)acetat der Formel (I.1) und Isomerengemische mit einem Gewichtsanteil von cis-(2-Isobutyl-4-methyl-tetrahydropyran-4-yl)acetat von wenigstens 90 Gew.-%, eignen sich zur Herstellung von Düften mit floralen und zitrusartigen Noten wie z. B. einer schwertlilienartigen Note, einer bergamotteartigen Note, einer linalylacetatartigen Note (Linalylacetat ist eine der Hauptkomponenten in Lavendelöl und Bergamotteöl), einer holzigen Note oder einer pfeffer/muskatartigen Note. Isomerenreines trans-(2-Isobutyl-4-methyl-tetrahydropyran-4-yl)acetat der Formel (I.2) und Isomerengemische mit einem Gewichtsanteil von trans-(2-lsobutyl-4-methyl-tetrahydropyran-4-yl)acetat von wenigstens 90 Gew.-%, eignen sich zur Herstellung von Düften mit einer jasminartigen Note, einer lederartigen Note oder einer kräftigen fruchtartigen Note. Diese Geruchseigenschaften unterscheiden sich deutlich von denen, die dem Gemisch gemäß der EP 0383446 zugeschrieben werden.
- Isomerenreines oder hoch isomerenangereichertes cis- oder trans-(2-Isobutyl-4-methyl-tetrahydropyran-4-yl)acetat ermöglicht die Bereitstellung facettenreicher sensorischer Profile durch Synergieeffekte mit anderen Riechstoffen. So werden z. B. bei der Kombination von isomerenreinem oder hoch isomerenangereichertem cis-(2-Isobutyl-4-methyl-tetrahydropyran-4-yl)acetat mit 2-substituierten 4-Hydroxy-4-methyl-tetrahydropyranen Geruchsprofile erzielt, die sich für florale Kompositionen mit Bergamotte- und Fruchtcharakter eignen. Bei der Kombination von isomerenreinem oder hoch isomerenangereichertem cis-(2-Isobutyl-4-methyl-tetrahydropyran-4-yl)acetat mit Linalylacetat werden Geruchsprofile erzielt, die ebenfalls sehr facettenreich sind.
- Die Herstellung von isomerenreinem oder hoch isomerenangereichertem cis- oder trans-(2-Isobutyl-4-methyl-tetrahydropyran-4-yl)acetat lässt sich leicht in die Herstellung von 2-Isobutyl-4-Hydroxy-4-methyl-tetrahydropyran integrieren. Die Herstellung gelingt beispielsweise ausgehend von 2-Isobutyl-4-Hydroxy-4-methyl-tetrahydropyran durch Veresterung und anschließende fraktionierte Destillation des Veresterungsprodukts.

Sofern im Folgenden nicht genauer angegeben, bezeichnen die Begriffe
cis-(2-Isobutyl-4-methyl-tetrahydropyran-4-yl)acetat (I.1) und trans-(2-Isobutyl-4-methyl-tetrahydropyran-4-yl)acetat (I.2)
alle Enantiomere in Reinform, optisch aktive Gemische der Enantiomeren dieser Verbindungen sowie optisch inaktive Racemate von cis-(I.1) und trans-(I.2). Sofern im Folgenden von cis- und trans-Diastereomeren der Verbindungen (I.1) oder (I.2) die Rede ist, wird jeweils nur eine der enantiomeren Formen abgebildet. Lediglich zur Veranschaulichung werden im Folgenden die Isomeren des 2-Isobutyl-4-methyl-tetrahydropyran-4-yl)acetats wiedergegeben:

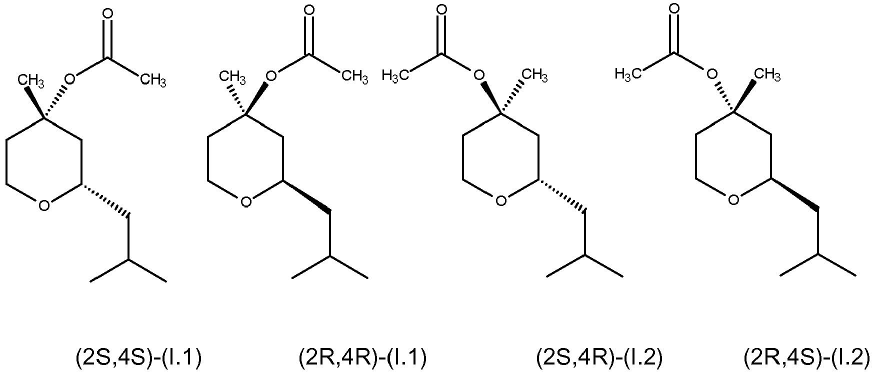

Die Verbindung der Formel (I.1) kann in stereoisomerenreiner Form oder als (2S,4S)- und (2R,4R)-Isomerengemisch eingesetzt werden.

Die Verbindung der Formel (I.2) kann in stereoisomerenreiner Form oder als (2S,4R)- und (2R,4S)-Isomerengemisch eingesetzt werden.

In einer speziellen Ausführungsform werden erfindungsgemäß optisch inaktive Racemate eingesetzt.

Im Folgenden wird ein Isomerengemisch aus cis-(2-Isobutyl-4-methyl-tetrahydropyran-4-yl)acetat (I.1) und trans-(2-Isobutyl-4-methyl-tetrahydropyran-4-yl)acetat (I.2), wobei der Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, als "hoch isomerenangereichertes cis-(2-Isobutyl-4-methyl-tetrahydropyran-4-yl)acetat" oder als "hoch isomerenangereichertes (I.1)" bezeichnet.

Im Folgenden wird als isomerenreines cis-(2-Isobutyl-4-methyl-tetrahydropyran-4-yl)acetat (I.1) das reine cis-Isomer bezeichnet. Ein isomerenreines cis-(2-Isobutyl-4-methyl-tetrahydropyran-4-yl)acetat (I.1) enthält kein (d. h. 0 Gew.-%) trans-(2-Isobutyl-4-methyl-tetrahydropyran-4-yl)acetat (I.2). Der Begriff "isomerenrein" bezieht sich in diesem Zusammenhang nur auf die cis/trans-Isomerie und nicht die Enantiomerie.

Im Folgenden wird ein Isomerengemisch aus cis-(2-Isobutyl-4-methyl-tetrahydropyran-4-yl)acetat (I.1) und trans-(2-Isobutyl-4-methyl-tetrahydropyran-4-yl)acetat (I.2), wobei der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, als "hoch isomerenangereichertes trans-(2-Isobutyl-4-methyl-tetrahydropyran-4-yl)acetat" oder als "hoch isomerenangereichertes (I.2)" bezeichnet.

Im Folgenden wird als isomerenreines trans-(2-Isobutyl-4-methyl-tetrahydropyran-4-yl)acetat (I.2) das reine trans-Isomer bezeichnet. Ein isomerenreines trans-(2-Isobutyl-4-methyl-tetrahydropyran-4-yl)acetat (I.2) enthält kein (d. h. 0 Gew.-%) cis-(2-Isobutyl-4-methyl-tetrahydropyran-4-yl)acetat (I.1). Der Begriff "isomerenrein" bezieht sich in diesem Zusammenhang nur auf die cis/trans-Isomerie und nicht die Enantiomerie.

Bevorzugt weist hoch isomerenangereichertes (I.1) einen Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) von 90 bis 99,999 Gew.-%, besonders bevorzugt von 92 bis 99,99 Gew.-%, insbesondere von 95 bis 99,9 Gew.-% auf.

Bevorzugt weist hoch isomerenangereichertes (I.2) einen Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) von 90 bis 99,999 Gew.-%, besonders bevorzugt von 92 bis 99,99 Gew.-%, insbesondere von 95 bis 99,9 Gew.-% auf.

Unter einer organoleptisch wirksamen Menge ist im Rahmen vorliegenden Erfindung eine solche Menge zu verstehen, die bei sachgemäßer Anwendung ausreicht, beim Anwender bzw. Verbraucher einen Dufteindruck hervorzurufen. Dabei handelt es sich bei Verwendung von isomerenreinem oder hoch isomerenangereichertem (I.1) speziell um den Eindruck eines angenehmen Geruchs nach Schwertlilien, Bergamotteöl oder Linalylacetat. Bei Verwendung von isomerenreinem oder hoch isomerenangereichertem (I.2) handelt es sich speziell um den Eindruck eines angenehmen Geruchs nach Jasmin.

### Herstellung von isomerenreinem oder hoch isomerenangereichertem (2-Isobutyl-4-methyl-tetrahydropyran-4-yl)acetat

Die Herstellung von isomerenreinem oder isomerenangereichertem (2-Isobutyl-4-methyl-tetrahydropyran-4-yl)acetat umfasst vorzugsweise eine Veresterung eines 2-Isopropyl-4-hydroxy-4-methyl-tetrahydropyrans der allgemeinen Formel (II). Die Überführung des 2-Isopropyl-4-hydroxy-4-methyl-tetrahydropyrans (II) in die entsprechenden Acetate der allgemeinen Formel (I) kann nach üblichen dem Fachmann bekannten Verfahren nach folgendem Schema erfolgen.

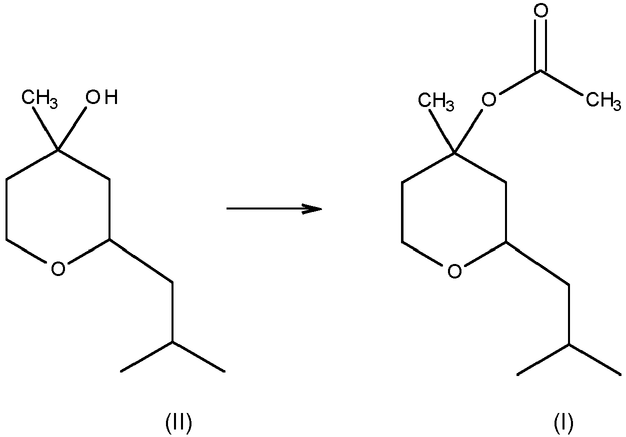

Bei der Veresterungsreaktion ändert sich die Konfiguration der eingesetzten Alkoholkomponente (II) in der Regel nicht. Zur Herstellung von isomerenreinem oder isomerenangereichertem (2-Isobutyl-4-methyl-tetrahydropyran-4-yl)acetat kann man somit in einer ersten Ausführungsform von einer isomerenreinen Alkoholkomponente (II) oder einer Alkoholkomponente (II) ausgehen, die bereits hinreichend hoch an dem gewünschten Isomer angereichert ist. In einer zweiten Ausführungsform wird zur Veresterung ein Isomerengemisch eingesetzt, das noch nicht hinreichend hoch an dem gewünschten Isomer angereichert ist und aus dem Produkt der Veresterung anschließend eine an dem gewünschten Isomeren hinreichend angereicherte Fraktion isoliert.

Ein weiterer Gegenstand der Erfindung ist somit ein Verfahren zur Herstellung von isomerenreinem (I.1) oder von isomerenreinem (I.2) oder von hoch isomerenangereicherte (I.1) oder von hoch isomerenangereichertem (I.2), bei dem man
i) ein Isomerengemisch aus cis-2-(2-Methylpropyl)-4-hydroxy-4-methyl-tetrahydropyran (II.1) und trans-2-(2-Methylpropyl)-4-hydroxy-4-methyl-tetrahydropyran (II.2) mit einer Verbindung der Formel CH₃C(=O)-X umsetzt, worin X für Cl, Br oder CH₃C(=O)O steht,
ii) gegebenenfalls das in Schritt i) erhaltene Reaktionsgemisch einer Auftrennung unter Erhalt wenigstens einer Fraktion unterzieht, die isomerenreines (I.1) oder isomerenreines (I.2) oder hoch isomerenangereichertes (I.1) oder hoch isomerenangereichertes (I.2) enthält.

Verfahren zur Herstellung des 2-Isopropyl-4-hydroxy-4-methyl-tetrahydropyrans (II), z. B. durch Umsetzung von Isoprenol mit Prenal und anschließende Hydrierung, sind dem Fachmann bekannt. Geeignete Verfahren sind z. B. in der WO 2011/147919, WO 2011/154330 und WO 2010/133473 beschrieben, worauf hier in vollem Umfang Bezug genommen wird.

Zur Veresterung kann das 2-Isopropyl-4-hydroxy-4-methyl-tetrahydropyran der allgemeinen Formel (II) mit Essigsäure oder einem geeigneten Derivat davon umgesetzt werden. Geeignete Derivate sind z. B. die Säurehalogenide und Säureanhydride. D. h. die Verbindung (II) wird umgesetzt mit einer Verbindung der Formel CH₃C(=O)-X, worin X für Cl, Br oder CH₃C(=O)O steht.

Die Veresterung erfolgt vorzugsweise in Gegenwart eines Veresterungskatalysators. Als Veresterungskatalysatoren können die dafür üblichen Katalysatoren eingesetzt werden, z. B. Mineralsäuren, wie Schwefelsäure und Phosphorsäure; organische Sulfonsäuren, wie Methansulfonsäure und p-Toluolsulfonsäure; amphotere Katalysatoren, insbesondere Titan-, Zinn (IV)- oder Zirkoniumverbindungen, wie Tetraalkoxytitane, z. B. Tetrabutoxytitan, und Zinn (IV)-oxid. Der Veresterungskatalysator wird in einer wirksamen Menge eingesetzt, die üblicherweise im Bereich von 0,05 bis 10 Gew.-%, vorzugsweise 0,1 bis 5 Gew.-%, bezogen auf die Summe von Säurekomponente (oder Anhydrid) und Alkoholkomponente, liegt.

Die Veresterung kann in der Regel bei Umgebungsdruck oder vermindertem oder erhöhtem Druck erfolgen. Bevorzugt wird die Veresterung bei Umgebungsdruck oder vermindertem Druck durchgeführt.

Die Veresterung kann in Abwesenheit eines zugesetzten Lösungsmittels oder in Gegenwart eines organischen Lösungsmittels durchgeführt werden. Falls die Veresterung in Gegenwart eines Lösungsmittels durchgeführt wird, handelt es sich dabei vorzugsweise um ein unter den Reaktionsbedingungen inertes organisches Lösungsmittel. Dazu gehören beispielsweise aliphatische Kohlenwasserstoffe, halogenierte aliphatische Kohlenwasserstoffe, aromatische und substituierte aromatische Kohlenwasserstoffe oder Ether. Bevorzugt ist das Lösungsmittel ausgewählt unter Pentan, Hexan, Heptan, Ligroin, Petrolether, Cyclohexan, Dichlormethan, Trichlormethan, Tetrachlormethan, Benzol, Toluol, Xylol, Chlorbenzol, Dichlorbenzolen, Dibutylether, THF, Dioxan und Mischungen davon.

Die Veresterung wird üblicherweise in einem Temperaturbereich von 0 bis 200 °C, bevorzugt 10 bis 150 °C, durchgeführt.

Die Veresterung kann in Abwesenheit oder in Gegenwart eines Inertgases erfolgen. Unter einem Inertgas wir in der Regel ein Gas verstanden, welches unter den gegebenen Reaktionsbedingungen keine Reaktionen mit den an der Reaktion beteiligten Edukten, Reagenzien, Lösungsmitteln oder den entstehenden Produkten eingeht. Dazu zählen z. B. Stickstoff oder Argon.

In einer speziellen Ausführungsform wird zur Herstellung eines isomerenreinen cis-(2-Isobutyl-4-methyl-tetrahydropyran-4-yl)acetats der Formel (I.1) oder eines isomerenreinen trans-(2-Isobutyl-4-methyl-tetrahydropyran-4-yl)acetat der Formel (I.2) oder eines hoch isomerenangereicherten (I.1) oder eines hoch isomerenangereicherten (I.2) ein Reaktionsgemisch der 2-Isobutyl-4-methyl-tetrahydropyran-4-yl)acetat-Synthese einer Auftrennung unter Erhalt einer Fraktion unterzogen, die isomerenreines cis-(I.1) oder isomerenreines trans-(I.2) oder eine an dem cis-Isomeren oder an dem trans-Isomeren angereicherte Fraktion isoliert.

Die üblichen zuvor genannten Verfahren zur Herstellung von 2-Isobutyl-4-methyl-tetrahydropyran-4-yl)acetat führen zu Reaktionsprodukten mit einem cis/trans-Verhältnis im Bereich von etwa 80 : 20 bis 20 : 80. Dementsprechend weist auch das Reaktionsgemisch der (2-Isobutyl-4-methyl-tetrahydropyran-4-yl)acetat-Synthese in der Regel ein cis/trans-Verhältnis im Bereich von etwa 80 : 20 bis 20 : 80 auf. Selbstverständlich kann zur Herstellung des 2-Isobutyl-4-methyl-tetrahydropyran-4-yl)acetats grundsätzlich ein 2-Isopropyl-4-hydroxy-4-methyl-tetrahydropyran mit beliebigem cis/trans-Verhältnis eingesetzt werden. Dabei kann es sich beispielsweise um technisch verfügbare Ströme aus der Synthese des 2-Isopropyl-4-hydroxy-4-methyl-tetrahydropyrans oder deren Aufarbeitung handeln.

Bevorzugt erfolgt die Isolierung einer Fraktion, die isomerenreines (I.1) oder isomerenreines (I.2) oder hoch isomerenangereichertes (I.1) oder hoch isomerenangereichertes (I.2) enthält (= Schritt ii)) mittels fraktionierter Destillation.

Geeignete Vorrichtungen zur destillativen Auftrennung umfassen Destillationskolonnen, wie Bodenkolonnen, die mit Glocken, Siebplatten, Siebböden, Packungen, Füllkörpern, Ventilen, Seitenabzügen, etc. ausgerüstet sein können, Verdampfer, wie Dünnschichtverdampfer, Fallfilmverdampfer, Zwangsumlaufverdampfer, Sambay-Verdampfer, etc., und Kombinationen davon.

Die Destillationskolonnen können trennwirksame Einbauten aufweisen, die vorzugsweise ausgewählt sind unter Trennböden, geordnete Packungen, z. B. Blech- oder Gewebepackungen, wie Sulzer Mellapak®, Sulzer BX, Montz B1 oder Montz A3 oder Kühni Rombopak, oder regellose Schüttungen von Füllkörpern, wie z. B. Dixon-Ringen, Raschig-Ringen, High-Flow-Ringen oder Raschig-Super-Ringen. Besonders bewährt haben sich geordnete Packungen, vorzugsweise Blech- oder Gewebepackungen, mit einer spezifischen Oberfläche von 100 bis 750 m²/m³, insbesondere 250 bis 500 m²/m³. Sie gestatten hohe Trennleistungen bei niedrigen Druckverlusten.

### Verwendung von isomerenreinem oder hoch isomerenangereichertem cis- oder trans-(2-Isobutyl-4-methyl-tetrahydropyran-4-yl)acetat

Das zuvor beschriebene Verfahren ermöglicht die Bereitstellung von isomerenreinem oder hoch isomerenangereichertem cis- oder trans-(2-Isobutyl-4-methyl-tetrahydropyran-4-yl)acetat.

Das erfindungsgemäße und erfindungsgemäß verwendete cis-(2-Isobutyl-4-methyl-tetrahydropyran-4-yl)acetat der Formel (I.1) weist einen Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) von 90 bis 100 Gew.-%, bevorzugt von 90 bis 99,999 Gew.-%, besonders bevorzugt von 92 bis 99,99 Gew.-%, insbesondere von 95 bis 99,9 Gew.-% auf.

Das erfindungsgemäße und erfindungsgemäß verwendete trans-(2-Isobutyl-4-methyl-tetrahydropyran-4-yl)acetat der Formel (I.2) weist einen Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) von 90 bis 100 Gew.-%, bevorzugt von 90 bis 99,999 Gew.-%, besonders bevorzugt von 92 bis 99,99 Gew.-%, insbesondere von 95 bis 99,9 Gew.-% auf.

Eine bevorzugte Ausführungsform ist die Verwendung von isomerenreinem oder hoch isomerenangreichertem (I.1) zur Herstellung eines Duftes mit einer schwertlilienartigen Note, einer bergamotteartigen Note, einer linalylacetatartigen Note, einer holzigen Note oder einer pfeffer/muskatartigen Note.

Eine weitere bevorzugte Ausführungsform ist die Verwendung von isomerenreinem oder hoch isomerenangreichertem (I.2) zur Herstellung eines Duftes mit einer jasminartigen Note, einer lederartigen Note oder einer kräftigen fruchtartigen Note.

Eine weitere bevorzugte Ausführungsform ist die Verwendung von isomerenreinem oder hoch isomerenangreichertem (I.1) zum teilweisen oder vollständigen Ersatz von Linalylacetat in einer Linalylacetat enthaltenden Duft- oder Aromastoffzusammensetzung oder in einem mit Linalylacetat parfümierten oder aromatisierten Produkt.

### Weitere Aromachemikalien:

Das erfindungsgemäße und erfindungsgemäß verwendete isomerenreine oder hoch isomerenangereicherte cis- oder trans-(2-Isobutyl-4-methyl-tetrahydropyran-4-yl)acetat wird in einer bevorzugten Ausführung der Erfindung in Kombination mit wenigstens einer weiteren, von den Verbindungen (I.1) und (I.2) verschiedenen Aromachemikalie eingesetzt. Bevorzugt handelt es sich bei den von den Verbindungen (I.1) und (I.2) verschiedenen Aromachemikalien um Riechstoffe. Erfindungsgemäße Riechstoffkompositionen enthalten dann beispielsweise 1, 2, 3, 4, 5, 6, 7, 8 oder mehr weitere Riechstoffe.

Weitere Aromastoffe und speziell Riechstoffe finden sich z. B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001.

Im Einzelnen seien genannt:
Extrakte aus natürlichen Rohstoffen wie etherischen Ölen, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z. B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und
3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyl-oxyacetaldehyd; 1-(1-Methoxy-propoxy)-(E/Z)-3-hexen; der aliphatischen Ketone und deren Oxime wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z. B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z. B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Geraniol; Nerol; Linalool; Lavandulol; Nerolidol; Farnesol; Tetrahydrolinalool; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6_Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7- Methoxy-3,7-dimethyloctanal; 2,6,1 0-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl- 2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal ; beta-Sinensal ; acetyliertes Cedernholzöl (Methylcedrylketon);
der cyclischen Alkohole wie z. B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z. B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6Trimethylcyclohexyl)hexan-3-ol;
der cyclischen und cycloaliphatischen Ether wie z. B. Cineol; Cedrylmethylether; Cyclododecylmethylether;1,1-Dimethoxycyclododecan; (Ethoxymethoxy)cyclododecan; alpha-Cedrenepoxid; 3a,6,6,9a Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z. B. 4-tert.-Butylcyclohexanon; 2,2,5-Trimethyl-5-pentylcyclopentanon; 2-Heptylcyclopentanon; 2-Pentylcyclo-pentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4 cyclopenta-decenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
der cycloaliphatischen Aldehyde wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-di methyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl) keton;
der Ester cyclischer Alkohole wie z. B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7 Methanooctahydro-5, bzw. 6-indenylacetat;
der Ester cycloaliphatischer Alkohole wie z. B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z. B. Benzylalkohol; 1-Phenylethylalkohol, 2-Phenylethylalkohol, 3-Phenylpropanol; 2-Phenylpropanol; 2-Phenoxyethanol;
2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3_phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z. B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropa-aldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)-propanal; 2-Methyl-3-(4-tert.-butylphenyl)propanal; 2-Methyl-3-(4-isobutylphenyl)-propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z. B. Acetophenon; 4 Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4 indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z. B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methyl-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z. B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z. B. 2,5-Dimethyl-4-hydroxy-2H-furan-3-on; 2-Ethyl-4-hydroxy-5-methyl-2Hfuran-3-on; 3-Hydroxy-2-methyl-4H-pyran-4-on; 2-Ethyl-3-hydroxy-4H-pyran-4-on;
der Lactone wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die weitere, von den Verbindungen (I.1) und (I.2) verschiedene Aromachemikalie ist vorzugsweise ausgewählt unter 2-Isobuty-4-methyl-tetrahydro-2H-pyran-4-ol, Linalylacetat, 7-Hydroxy-3,7-dimethyloctanal, 4-Isopropyl-cyclohexylmethanol, 4-(Octahydro-4,7-methano-5H-inden-5-yliden)butanal, 4-(4-Hydroxy-4-methylpentyl)-3-cyclohexen-1-carboxaldehyd, 3-(4-Hydroxy-4-methylpentyl)-3-cyclohexen-1-carboxaldehyd, 2,5,7,7-Tetramethyloctanal, 2-Methyl-3-(4-tert.-butylphenyl)propanal, 3-(4-tert.-butylphenyl)propanal, Linalool, Ethyllinalool, Tetrahydrolinalool und 2-Methyl-4-phenyl-2-butanol).

### Duft- oder Aromastoffzusammensetzung

Ein weiterer Gegenstand der Erfindung ist eine Duft- oder Aromastoffzusammensetzung, enthaltend
a) isomerenreines cis-(2-Isobutyl-4-methyl-tetrahydropyran-4-yl)acetat der Formel (I.1) oder isomerenreines trans-(2-Isobutyl-4-methyl-tetrahydropyran-4-yl)acetat der Formel (I.2) oder ein Isomerengemisch aus cis-(2-Isobutyl-4-methyl-tetrahydropyran-4-yl)acetat (I.1) und trans-(2-Isobutyl-4-methyl-tetrahydropyran-4-yl)acetat (I.2), wobei der Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, oder der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt,
b) gegebenenfalls wenigstens eine weitere, von den Verbindungen (I.1) und (I.2) verschiedene Aromachemikalie, und
c) gegebenenfalls wenigstens ein Verdünnungsmittel,
mit der Maßgabe, das die Zusammensetzung wenigstens eine Komponente b) oder c) enthält.

Bevorzugt enthält die erfindungsgemäße Duft- oder Aromastoffzusammensetzung die Komponente a) in einem Gewichtsanteil von 0,1 to 95 Gew.-%, besonders bevorzugt von 0,1 to 90 Gew.-%, insbesondere von 0,1 bis 80 Gew.-%, bezogen auf das Gesamtgewicht der Zusammensetzung.

Bevorzugter enthält die erfindungsgemäße Duft- oder Aromastoffzusammensetzung die Komponente a) in einem Gewichtsanteil von 0,1 bis 70 Gew.-%, stärker bevorzugt 1 bis 50 Gew.-%, bezogen auf das Gesamtgewicht der Zusammensetzung. In einer speziellen Ausführungsform enthält die erfindungsgemäße Duft- oder Aromastoffzusammensetzung die Komponente a) in einem Gewichtsanteil von 2 bis 30 Gew.-%, spezieller 3 bis 15 Gew.-%, bezogen auf das Gesamtgewicht der Zusammensetzung.

In einer bevorzugten Ausführungsform enthält die erfindungsgemäße Duft- oder Aromastoffzusammensetzung die Komponente a) als alleinige Aromachemikalie.

In einer weiteren bevorzugten Ausführungsform enthält die erfindungsgemäße Duft- oder Aromastoffzusammensetzung wenigstens eine weitere, von den Verbindungen (I.1) und (I.2) verschiedene Aromachemikalie b).

Geeignete weitere Aromachemikalien b) sind die zuvor genannten, worauf hier in vollem Umfang Bezug genommen wird.

Bevorzugt liegt das Gewichtsmengenverhältnis von Komponente a) zu Komponente b) in einem Bereich von 100 : 1 bis 1 : 100, besonders bevorzugt von 50 : 1 bis 1 : 50.

Die Duft- oder Aromastoffzusammensetzung kann gegebenenfalls wenigstens ein Verdünnungsmittel c) enthalten. Geeignete Verdünnungsmittels können einzeln oder als Gemisch von 2 oder mehr als 2 Verdünnungsmitteln eingesetzt werden. Geeignete Verdünnungsmittel sind solche, wie sie üblicherweise als Lösungsmittel für Duft- oder Aromastoffe eingesetzt werden.

Bevorzugt enthält die Duft- oder Aromastoffzusammensetzung als Verdünnungsmittel c) wenigstens eine Verbindung, die bei 20 °C und 1013 mbar flüssig ist.

Bevorzugt haben die Verbindungen der Komponente a) in der Komponente c) eine Löslichkeit bei 20 °C von wenigstens 0,1 mg/ml, besonders bevorzugt von wenigstens 0,5 mg/ml. Bevorzugt haben falls vorhanden, die Verbindungen der Komponente b) in der Komponente c) eine Löslichkeit bei 20 °C von wenigstens 0,1 mg/ml, besonders bevorzugt von wenigstens 0,5 mg/ml.

Bevorzugt ist die Komponente c) ausgewählt unter aliphatischen und cycloaliphatischen Monoalkoholen, Polyolen, offenkettigen aliphatische Ethern, cyclischen Ethern, Polyolmono- und -polyethern, Estern und Mischungen davon.

Geeignete aliphatische und cycloaliphatische Monoalkohole sind z. B. Ethanol, n-Propanol, Isopropanol n-Butanol, sec.-Butanol, tert.-Butanol und Cyclohexanol.

Geeignete Polyole sind Ethylenglycol, Propylenglycol, 1,2-Butylenglycol, Diethylenglycol, Dipropylenglycol oder Glycerin.

Geeignete offenkettige aliphatische Ether und cyclische Ether sind z. B. Diethylether, Dipropylether, Diisopropylether, Methyl-tert-butyl-ether, Tetrahydrofuran, tetrahydropyran, 1,4-Dioxan oder Morpholin.

Geeignete Polyolmono- und -polyether sind z. B. Ethylenglycolmonomethylether, Ethylenglycoldimethylether, Ethylenglycolmonoethylether, Ethylenglycoldiethylether, Propylenglycolmonoethylether, Propylenglycoldiethylether oder Diethylenglycolmonoethylether.

Geeignete Ester sind Ethylacetat, Methylacetat, Propylacetat, Isopropylacetat, n-Butylacetat, sec-Butylacetat, tert-Butylacetat, Isobutylacetat, Isoamylacetat, Ethylbutyrate, Ethyllactat, Diethylcarbonat, Ethylencarbonate, Propylencarbonat, Triethylcitrat, Isopropylmyristat, Diethylphthalat, Dialkylesters von 1,2-Cyclohexandicarbonsäure, speziell 1,2-Cyclohexandicarbonsäurediisononylester (Hexamoll ® DINCH, BASF SE), etc.

### Parfümiertes oder aromatisiertes Produkt

Erfindungsgemäßes und erfindungsgemäß verwendetes isomerenreines oder hoch isomerenangereichertes cis- oder trans-(2-Isobutyl-4-methyl-tetrahydropyran-4-yl)-acetat kann in eine Reihe von Produkten eingearbeitet bzw. auf solche Produkte appliziert werden.

Erfindungsgemäße Riechstoffe können bei der Herstellung parfümierter Artikel eingesetzt. Die olfaktorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäßen Riechstoffe unterstreichen ihre besondere Eignung für die genannten Einsatzzwecke. Die positiven Eigenschaften tragen dazu bei, dass die erfindungsgemäß verwendeten Riechstoffe und die erfindungsgemäßen Riechstoffkompositionen besonders bevorzugt in Parfümerzeugnissen, Körperpflegeprodukten, Hygieneartikeln, Textilwaschmittel sowie in Reinigungsmitteln für feste Oberflächen eingesetzt werden.

Der parfümierte Artikel ist z. B. ausgewählt unter Parfümerzeugnissen, Körperpflegeprodukten, Hygieneartikeln, Textilwaschmitteln und Reinigungsmitteln für feste Oberflächen. Bevorzugte erfindungsgemäße parfümierte Artikel sind weiterhin ausgewählt unter:
Parfümerzeugnissen, ausgewählt unter Parfüm-Extrakten, Eau de Parfums, Eau de Toilettes, Eau de Colognes, Eau de Solide, Extrait Parfum, Luftverbesserern in flüssiger, gelartiger oder auf einem festen Träger aufgebrachter Form, Aerosolsprays, Duftreinigern und -ölen;
Körperpflegeprodukten, ausgewählt unter Rasierwässern, Pre-shave-Produkten, Splash-Colognes, festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-ÖI-in-Wasser-Typ wie z. B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und - lotionen, Haarpflegeprodukten wie z. B. Haarsprays, Haargelen, festigende Haarlotionen, Haarspülungen, Haarshampoo, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes, Produkten der dekorativen Kosmetik wie z. B. Lidschatten, Nagellacke, Make-ups, Lippenstifte, Mascara, Zahnpasta, Zahnseide;
Hygieneartikeln, ausgewählt unter Kerzen, Lampenölen, Räucherstäbchen, Insektiziden, Repellentien, Treibstoffen, Rostentfernern, parfümierten Erfrischungstüchern, Achselpads, Babywindeln, Damenbinden, Toilettenpapier, Kosmetiktüchern, Taschentüchern, Spülmaschinendeo;
Reinigungsmitteln für feste Oberflächen, ausgewählt unter parfümierten sauren, alkalischen und neutralen Reinigungsmitteln, wie z. B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Wachsen und Polituren wie Möbelpolituren, Fußbodenwachsen, Schuhcremes, Desinfektionsmitteln, Oberflächendesinfektionsmitteln und Sanitärreinigern, Bremsenreinigern, Rohrreinigern, Entkalkern, Grill- und Backofenreinigern, Algen- und Moosentfernern, Schimmelentfernern, Fassadenreinigungsmitteln;
Textilwaschmitteln, ausgewählt unter flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten.

Einem weiteren Aspekt zufolge sind die erfindungsgemäß verwendeten Riechstoffe und die erfindungsgemäßen Riechstoffkompositionen für den Einsatz in tensidhaltigen parfümierten Artikeln geeignet. Gesucht werden nämlich - insbesondere für die Parfümierung von tensidhaltigen Formulierungen wie z. B. Reinigungsmittel (insbesondere Geschirrspülmittel und Allzweckreiniger) - häufig Riechstoffe und/oder Riechstoffkompositionen mit einer Rosenkopfnote und ausgeprägter Natürlichkeit.

Einem weiteren Aspekt zufolge können erfindungsgemäß verwendete Riechstoffe und erfindungsgemäße Riechstoffkompositionen als Mittel zum Versehen von (a) Haaren oder (b) textilen Fasern mit der Geruchsnote rosig verwendet werden.

Die erfindungsgemäß zu verwendenden Riechstoffe und erfindungsgemäße Riechstoffkompositionen eignen sich daher besonders gut für den Einsatz in tensidhaltigen parfümierten Artikeln.

Bevorzugt ist es, wenn der parfümierte Artikel einer der Folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos Rasierseifen, Rasierschäumen, Badeölen, kosmetischen Emulsionen vom Öl-in-Wasser-, vom Wasser-in-Öl- und vom Wasser-in-Öl-in-Wasser-Typ wie z. B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z. B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes, Produkten der dekorativen Kosmetik.

Inhaltstoffe, mit denen erfindungsgemäß verwendete Riechstoffe oder erfindungsgemäße Riechstoffkompositionen vorzugsweise kombiniert werden können, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, a-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Einem weiteren Aspekt zufolge verwendet man die Riechstoffe bei der Herstellung der parfümierten Artikel in flüssiger Form, unverdünnt oder mit einem Lösungsmittel verdünnt oder in Form einer Riechstoffkomposition. Geeignete Lösungsmittel hierfür sind die zuvor als Komponente c) genannt. Auf diese wird in vollem Umfang Bezug genommen.

Die in den erfindungsgemäßen parfümierten Artikeln enthaltenen Riechstoffe und/oder Riechstoffkompositionen können dabei in einer Ausführungsform an einem Trägerstoff absorbiert sein, der sowohl für eine feine Verteilung des Riechstoffs oder der Riechstoffkomposition im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäß verwendeten Riechstoffe und die erfindungsgemäßen Riechstoffkompositionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden. Die Eigenschaften können durch sogenanntes "Coaten" mit geeigneten Materialien im Hinblick auf eine gezieltere Duftfreisetzung weiter optimiert werden, wozu vorzugsweise wachsartige Kunststoffe wie z. B. Polyvinylalkohol verwendet werden.

Die Mikroverkapselung kann beispielsweise durch das sogenannte Koazervationsverfahren mit Hilfe von Kapselmaterialien, z. B. aus polyurethanartigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z. B. durch Eintragen von Dispersionen von Riechstoffkompositionen und Cyclodextrinen oder Harnstoffderivaten in ein geeignetes Lösungsmittel, z. B. Wasser, hergestellt werden. Extrusionsprodukte können durch Verschmelzen erfindungsgemäß verwendeter Riechstoffe und erfindungsgemäßer Riechstoffkompositionen mit einem geeigneten wachsartigen Stoff und durch Extrusion mit nachfolgender Erstarrung, ggf. in einem geeigneten Lösungsmittel, z. B. Isopropanol, hergestellt werden.

Die folgenden Beispiele dienen der Verdeutlichung der Erfindung, ohne sie in irgendeiner Weise zu beschränken.

### BEISPIELE

### Synthese:

Die Veresterung eines Isomerengemischs aus cis-2-(2-Methylpropyl)-4-hydroxy-4-methyl-tetrahydropyran (II.1) und trans-2-(2-Methylpropyl)-4-hydroxy-4-methyl-tetrahydropyran (II.2) erfolgte durch Umsetzung mit Essigsäureanhydrid und katalytischen Mengen von Methansulfonsäure nach einem dem Fachmann bekannten Verfahren. Ein solches Verfahren ist in der EP 0383446 A2 beschrieben.

### Destillation:

In einer Labor-Batch-Kolonne wurden 1,4 kg Rohprodukt aus der Pyranolacetatsynthese, enthaltend ca. 71 GC-FI.% cis-Pyranolacetat (mittels Gaschromatographie ermittelte Flächen-%-Werte), ca. 19 GC-FI.% trans-Pyranolacetat, ca. 6 GC-FI.% restliches Lösungsmittel und ca. 4 GC-FI.% nicht umgesetztes 2-(2-Methylpropyl)-4-hydroxy-4-methyl-tetrahydropyran, destilliert. Die verwendete Labor-Batch-Kolonne bestand aus einer 1,6 L Siedeblase, welche elektrisch beheizbar ist. Auf die Blase ist ein 43 mm breiter Verstärkungsteil mit 1,4 m Packung (Montz A3-1000) aus Glas aufgesetzt, was in etwa 20 theoretischen Stufen entspricht. Auf der Kolonne befindet sich ein Kondensator, welcher bei 15 °C betrieben wurde. Das Kondensat wurde beim Herunterfließen auf die Kolonne durch einen Rücklaufteiler in einen Rücklaufstrom und einen Destillatstrom aufgeteilt.

Die Fraktionierung wurde bei einem Kopfdruck von 4 mbar und einem konstantem Rücklaufverhältnis von 10 : 1 durchgeführt. Dabei wurden 13 Fraktionen (siehe Diagramm 1) gewonnen. Die Kopftemperatur während der gesamten Destillation lag im Bereich von 83 bis 92 °C. In Diagramm 1 steht RT für Retentionszeit in Minuten

**Diagramm 1: Zusammensetzung der Fraktionen in GC-FI.% der Destillation von Pyranolacetat**

| GC-Analyse | Aufteilung | | | Toluol RT 4,9 | trans-Pyranolacetat RT 23,3 | cis-Pyranolacetat RT 26,9 | Summe Pyranolacetat | Pyranol trans RT 27,1 | Pyranol cis RT 28,5 | Rest |
|---|---|---|---|---|---|---|---|---|---|---|
| | g | % | Σ% | Flächen % | | | | | | |
| Einsatz | 1417 | 100,00 | 0,00 | 6,26 | 18,49 | 71,22 | 89,71 | 1,90 | 1,83 | 0,30 |
| Kühlfalle | 70 | 4,94 | 4,94 | 94,35 | 0,69 | 0,02 | 0,71 | 0,05 | 0,02 | 4,87 |
| Fraktion 1 | 88 | 6,21 | 11,15 | 0 | 89,8 | 0,08 | 89,88 | 9,04 | 0,40 | 0,68 |
| Fraktion 2 | 94 | 6,63 | 17,78 | 0 | 88,39 | 0,02 | 88,41 | 8,85 | 2,52 | 0,22 |
| Fraktion 3 | 58 | 4,09 | 21,88 | 0 | 78,8 | 2,12 | 80,92 | 9,07 | 9,58 | 0,43 |
| Fraktion 4 | 95 | 6,70 | 28,58 | 0 | 36,11 | 45,08 | 81,19 | 4,86 | 13,73 | 0,22 |
| Fraktion 5 | 96 | 6,77 | 35,36 | 0 | 12,61 | 82,81 | 95,42 | 1,26 | 3,25 | 0,07 |
| Fraktion 6 | 52 | 3,67 | 39,03 | 0 | 9,2 | 87,42 | 96,62 | 0,92 | 2,38 | 0,08 |
| Fraktion 7 | 109 | 7,69 | 46,72 | 0 | 2 | 97,18 | 99,18 | 0,22 | 0,59 | 0,01 |
| Fraktion 8 | 97 | 6,85 | 53,56 | 0 | 0,57 | 99,07 | 99,64 | 0,13 | 0,21 | 0,02 |
| Fraktion 9 | 114 | 8,05 | 61,61 | 0 | 0,18 | 99,68 | 99,86 | 0,00 | 0,12 | 0,02 |
| Fraktion 10 | 110 | 7,76 | 69,37 | 0 | 0,01 | 99,95 | 99,96 | 0,00 | 0,02 | 0,02 |
| Fraktion 11 | 92 | 6,49 | 75,86 | 0 | 0 | 99,97 | 99,97 | 0,00 | 0,01 | 0,02 |
| Fraktion 12 | 121 | 8,54 | 84,40 | 0 | 0,01 | 99,95 | 99,96 | 0,00 | 0,01 | 0,03 |
| Fraktion 13 | 95 | 6,70 | 91,11 | 0 | 0 | 99,97 | 99,97 | 0,00 | 0,00 | 0,03 |
| Sumpf | 119 | 8,40 | 99,51 | 0 | 0 | 98,06 | 98,06 | 0,16 | 0,01 | 1,77 |
| Summe | 1410,0 | | | | | | | | | |
| Verlust | 7,0 | | | | | | | | | |
| Wiederfindung | 99,50% | | | | | | | | | |

### Olfaktorische Bewertung der hoch isomerenangereicherten Komponenten:

High-cis-Pyranolacetat (> 99 % cis-(2-Isobutyl-4-methyl-tetrahydropyran-4-yl)acetat der Formel (1.1)): schwertlilienartige Note, eine kräftige, klare bergamotteartige Note, eine linalylacetatartige Note, eine holzige Note sowie eine pfeffer/muskatartige Note.

High-trans-Pyranolacetat (> 98 % trans-(2-Isobutyl-4-methyl-tetrahydropyran-4-yl)-acetat der Formel (I.2)): jasminartige Note, eine lederartige Note und eine kräftige fruchtartige Note.

Im Vergleich dazu weist eine Mischung aus 75 % cis-(2-Isobutyl-4-methyl-tetrahydropyran-4-yl)acetat (I.1) und 25 % trans-(2-Isobutyl-4-methyl-tetrahydropyran-4-yl)acetat (I.2) folgende olfaktorische Eigenschaften auf: blumig, Linalylacetat, Agrumen. Im Hinblick darauf sind sowohl die olfaktorischen Eigenschaften des High-cis-Pyranolacetats als auch des High-trans-Pyranolacetats überraschend.

## Patentansprüche

1. Verwendung von isomerenreinem cis-(2-Isobutyl-4-methyl-tetrahydropyran-4-yl)acetat der Formel (I.1) oder von isomerenreinem trans-(2-Isobutyl-4-methyl-tetrahydropyran-4-yl)acetat der Formel (I.2) oder eines Isomerengemischs aus cis-(2-Isobutyl-4-methyl-tetrahydropyran-4-yl)acetat (I.1) und trans-(2-Isobutyl-4-methyl-tetrahydropyran-4-yl)acetat (I.2) 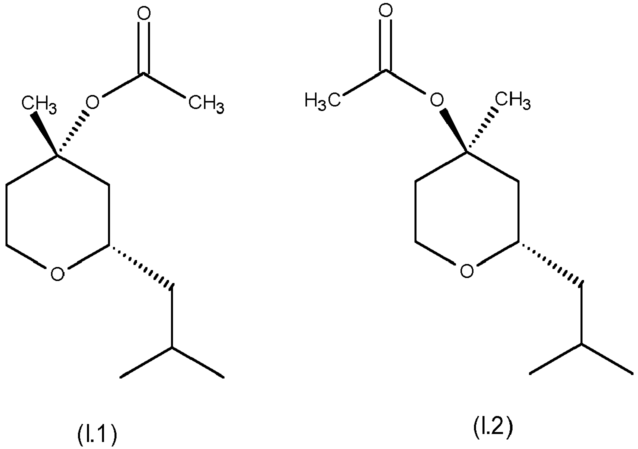
wobei bei Verwendung eines Isomerengemischs
der Gewichtsanteil von cis-(2-Isobutyl-4-methyl-tetrahydropyran-4-yl)acetat (I.1) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, oder
der Gewichtsanteil von trans-(2-Isobutyl-4-methyl-tetrahydropyran-4-yl)acetat (I.2) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt,
als Aromachemikalie.

2. Verwendung nach Anspruch 1 in Mitteln, ausgewählt unter Parfüms, Wasch- und Reinigungsmitteln, kosmetischen Mitteln, Körperpflegemitteln, Hygieneartikeln, Produkten für die Mund- und Zahnhygiene, Duftspendern und Duftstoffen.

3. Verwendung nach Anspruch 1 oder 2 von isomerenreinem cis-(2-Isobutyl-4-methyl-tetrahydropyran-4-yl)acetat der Formel (I.1) oder eines Isomerengemischs, wobei der Gewichtsanteil von cis-(2-Isobutyl-4-methyl-tetrahydropyran-4-yl)acetat (I.1) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, zur Herstellung eines Duftes mit einer floralen, holzigen oder zitrusartigen Note.

4. Verwendung nach Anspruch 1 oder 2 von isomerenreinem trans-(2-Isobutyl-4-methyl-tetrahydropyran-4-yl)acetat der Formel (I.2) oder eines Isomerengemischs, wobei der Gewichtsanteil von trans-(2-Isobutyl-4-methyl-tetrahydropyran-4-yl)acetat (I.2) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, zur Herstellung eines Duftes mit einer jasminartigen Note, einer lederartigen Note oder einer kräftigen fruchtartigen Note.

5. Verwendung nach einem der vorhergehenden Ansprüche, wobei die Verbindung der Formel (I.1) in stereoisomerenreiner Form oder als (2S,4S)- und (2R,4R)-Isomerengemisch eingesetzt wird.

6. Verwendung nach einem der vorhergehenden Ansprüche, wobei die Verbindung der Formel (I.2) in stereoisomerenreiner Form oder als (2S,4R)- und (2R,4S)-Isomerengemisch eingesetzt wird.

7. Verfahren zur Herstellung von isomerenreinem cis-(2-Isobutyl-4-methyl-tetrahydropyran-4-yl)acetat der Formel (I.1) oder von isomerenreinem trans-(2-Isobutyl-4-methyl-tetrahydropyran-4-yl)acetat der Formel (I.2) oder eines Isomerengemischs aus (I.1) und (I.2), wobei der Gewichtsanteil von (I.1) oder der Gewichtsanteil von (I.2) in dem Isomerengemisch, bezogen auf das Gesamtgewicht von (I.1) und (I.2), in einem Bereich von 90 Gew.-% bis weniger als 100 Gew.-% liegt,
bei dem man
i) ein Isomerengemisch aus cis-2-(2-Methylpropyl)-4-hydroxy-4-methyl-tetrahydropyran (II.1) und trans-2-(2-Methylpropyl)-4-hydroxy-4-methyl-tetrahydropyran (II.2) 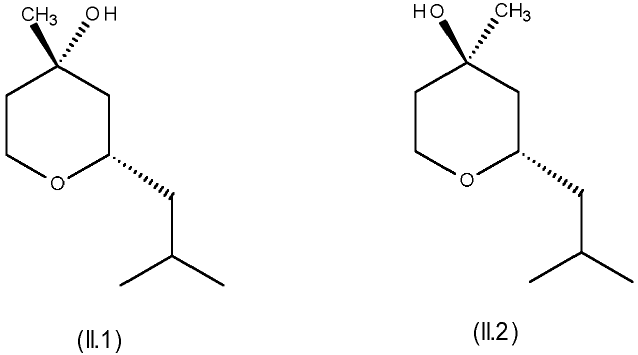 mit einer Verbindung der Formel CH₃C(=O)-X umsetzt, worin X für Cl, Br oder CH₃C(=O)O steht,
ii) gegebenenfalls das in Schritt i) erhaltene Reaktionsgemisch einer Auftrennung unter Erhalt wenigstens einer Fraktion unterzieht, die isomerenreines (I.1) oder isomerenreines (I.2) oder ein Isomerengemisch aus (I.1) und (I.2) enthält, wobei der Gewichtsanteil von (I.1) oder der Gewichtsanteil von (I.2) in dem Isomerengemisch, bezogen auf das Gesamtgewicht von (I.1) und (I.2), in einem Bereich von 90 Gew.-% bis weniger als 100 Gew.-% liegt.

8. Isomerenreines cis-(2-Isobutyl-4-methyl-tetrahydropyran-4-yl)acetat der Formel (I.1) oder Isomerengemische aus cis-(2-Isobutyl-4-methyl-tetrahydropyran-4-yl)acetat (I.1) und trans-(2-Isobutyl-4-methyl-tetrahydropyran-4-yl)acetat (I.2) worin der Gewichtsanteil von cis-(2-Isobutyl-4-methyl-tetrahydropyran-4-yl)acetat (I.1) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt.

9. Isomerenreines trans-(2-Isobutyl-4-methyl-tetrahydropyran-4-yl)acetat der Formel (I.2) oder Isomerengemische aus cis-(2-Isobutyl-4-methyl-tetrahydropyran-4-yl)acetat (I.1) und trans-(2-Isobutyl-4-methyl-tetrahydropyran-4-yl)acetat (I.2) worin der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt.

10. Duft- oder Aromastoffzusammensetzung, enthaltend
a) isomerenreines cis-(2-Isobutyl-4-methyl-tetrahydropyran-4-yl)acetat der Formel (I.1) oder isomerenreines trans-(2-Isobutyl-4-methyl-tetrahydropyran-4-yl)acetat der Formel (I.2) oder ein Isomerengemisch aus cis-(2-Isobutyl-4-methyl-tetrahydropyran-4-yl)acetat (I.1) und trans-(2-Isobutyl-4-methyl-tetrahydropyran-4-yl)acetat (I.2), wobei der Gewichtsanteil von cis-(2-Isobutyl-4-methyl-tetrahydropyran-4-yl)acetat (I.1) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, oder der Gewichtsanteil von trans-(2-Isobutyl-4-methyl-tetrahydropyran-4-yl)acetat (I.2) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt,
b) gegebenenfalls wenigstens eine weitere, von den Verbindungen (I.1) und (I.2) verschiedene Aromachemikalie, und
c) gegebenenfalls wenigstens ein Verdünnungsmittel,
mit der Maßgabe, das die Zusammensetzung wenigstens eine Komponente b) oder c) enthält.

11. Zusammensetzung nach Anspruch 10, enthaltend die Komponente a) in einem Gewichtsanteil von 0,1 bis 70 Gew.-%, bevorzugt 1 bis 50 Gew.-%, bezogen auf das Gesamtgewicht der Zusammensetzung.

12. Verfahren zum Verleihen und/oder Verstärken eines Geruchs oder Geschmacks eines Produkts mit einer schwertlilienartigen Note, einer bergamotteartigen Note, einer linalylacetatartigen Note, einer holzigen Note oder einer pfeffer/muskatartigen Note, bei dem das Produkt mit einer organoleptisch wirksamen Menge eines isomerenreinen cis-(2-Isobutyl-4-methyl-tetrahydropyran-4-yl)acetats der Formel (I.1) oder einem Isomerengemischs, wobei der Gewichtsanteil von cis-(2-Isobutyl-4-methyl-tetrahydropyran-4-yl)acetat (I.1) bezogen auf das Gesamtgewicht von cis- 2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, in Kontakt gebracht wird.

13. Verfahren zum Verleihen und/oder Verstärken eines Geruchs oder Geschmacks eines Produkts mit einer jasminartigen Note, einer lederartigen Note oder einer kräftigen fruchtartigen Note, bei dem das Produkt mit einer organoleptisch wirksamen Menge eines isomerenreinen trans-(2-Isobutyl-4-methyl-tetrahydropyran-4-yl)acetats der Formel (I.2) oder einem Isomerengemischs, wobei der Gewichtsanteil von trans-(2-Isobutyl-4-methyl-tetrahydropyran-4-yl)acetat (I.2) bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, in Kontakt gebracht wird.

14. Parfümiertes oder aromatisiertes Produkt, umfassend eine organoleptisch wirksame Menge an isomerenreinem cis-(2-Isobutyl-4-methyl-tetrahydropyran-4-yl)acetat der Formel (I.1) oder an isomerenreinem trans-(2-Isobutyl-4-methyl-tetrahydropyran-4-yl)acetat der Formel (I.2) oder eines Isomerengemischs aus cis-(2-Isobutyl-4-methyl-tetrahydropyran-4-yl)acetat (I.1) und trans-(2-Isobutyl-4-methyl-tetrahydropyran-4-yl)acetat (I.2), wie in einem der Ansprüche 1 bis 6 definiert, oder umfassend eine organoleptisch wirksame Menge einer Duft- oder Aromastoffzusammensetzung, wie in einem der Ansprüche 10 oder 11 definiert.

15. Verwendung von isomerenreinem cis-(2-Isobutyl-4-methyl-tetrahydropyran-4-yl)acetat der Formel (I.1) oder eines Isomerengemischs, wobei der Gewichtsanteil von cis-(2-Isobutyl-4-methyl-tetrahydropyran-4-yl)acetat (I.1), bezogen auf das Gesamtgewicht von cis-2-Isobutyl-4-methyl-tetrahydropyran-4-yl)acetat (I.1) und trans-(2-Isobutyl-4-methyl-tetrahydropyran-4-yl)acetat (I.2) in einem Bereich von 90 Gew.-% bis weniger als 100 Gew.-% liegt, zum teilweisen oder vollständigen Ersatz von Linalylacetat in einer Linalylacetat enthaltenden Duft- oder Aromastoffzusammensetzung oder in einem mit Linalylacetat parfümierten oder aromatisierten Produkt.

## Claims

1. The use of isomerically pure cis-(2-isobutyl-4-methyltetrahydropyran-4-yl) acetate of the formula (I.1) or of isomerically pure trans-(2-isobutyl-4-methyltetrahydropyran-4-yl) acetate of the formula (I.2) or of an isomer mixture of cis-(2-isobutyl-4-methyltetrahydropyran-4-yl) acetate (I.1) and trans-(2-isobutyl-4-methyltetrahydropyran-4-yl) acetate (1.2) 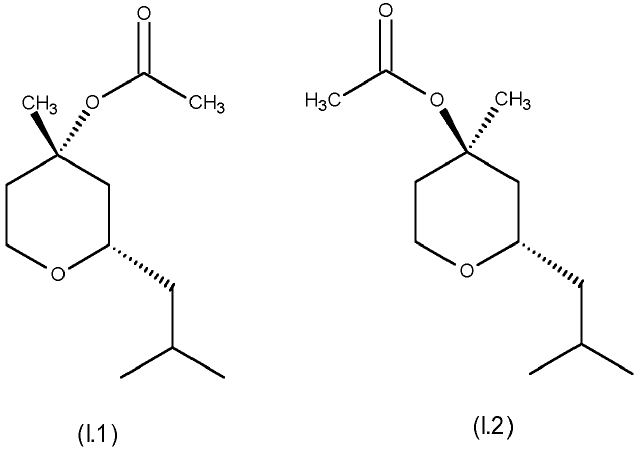
wherein, when using an isomer mixture,
the weight fraction of cis-(2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2), is in a range from 90% by weight to less than 100% by weight, or
the weight fraction of trans-(2-isobutyl-4-methyltetrahydropyran-4-yl) acetate (I.2), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight,
as aroma chemical.

2. The use according to claim 1 in compositions selected from perfumes, detergents and cleaners, cosmetic compositions, bodycare compositions, hygiene articles, products for oral and dental hygiene, scent dispensers and fragrances.

3. The use according to claim 1 or 2 of isomerically pure cis-(2-isobutyl-4-methyltetrahydropyran-4-yl) acetate of the formula (I.1) or of an isomer mixture, where the weight fraction of cis- (2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2), is in a range from 90% by weight to less than 100% by weight, for producing a scent with a floral, woody or citrus-like note.

4. The use according to claim 1 or 2 of isomerically pure trans-(2-isobutyl-4-methyltetrahydropyran-4-yl) acetate of the formula (I.2) or of an isomer mixture, where the weight fraction of trans-(2-isobutyl-4-methyltetrahydropyran-4-yl) acetate (I.2), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight, for producing a scent with a jasmine-like note, a leather-like note or a strong fruit-like note.

5. The use according to any one of the preceding claims, where the compound of the formula (I.1) is used in stereoisomerically pure form or as (2S, 4S)- and (2R,4R)-isomer mixture.

6. The use according to any one of the preceding claims, where the compound of the formula (I.2) is used in stereoisomerically pure form or as (2S, 4R)- and (2R,4S)-isomer mixture.

7. A process for the preparation of isomerically pure cis-(2-isobutyl-4-methyltetrahydropyran-4-yl) acetate of the formula (I.1) or of isomerically pure trans-(2-isobutyl-4-methyltetrahydropyran-4-yl) acetate of the formula (I.2) or of an isomer mixture of (I.1) and (1.2), where the weight fraction of (I.1) or the weight fraction of (I.2) in the isomer mixture, based on the total weight of (I.1) and (I.2), is in a range from 90% by weight to less than 100% by weight,
in which
i) an isomer mixture of cis-2-(2-methylpropyl)-4-hydroxy-4-methyltetrahydropyran (II.1) and trans-2-(2-methylpropyl)-4-hydroxy-4-methyltetrahydropyran (II.2) 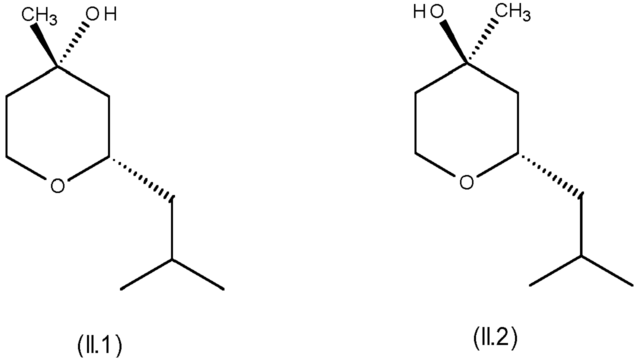 is reacted with a compound of the formula CH₃C(=O)-X, in which X is Cl, Br or CH₃C(=O)O,
ii) optionally the reaction mixture obtained in step i) is subjected to a separation to give at least one fraction which comprises isomerically pure (I.1) or isomerically pure (I.2) or an isomer mixture of (I.1) and (I.2), where the weight fraction of (I.1) or the weight fraction of (I.2) in the isomer mixture, based on the total weight of (I.1) and (I.2), is in a range from 90% by weight to less than 100% by weight.

8. An isomerically pure cis-(2-isobutyl-4-methyltetrahydropyran-4-yl) acetate of the formula (I.1) or an isomer mixture of cis-(2-isobutyl-4-methyltetrahydropyran-4-yl) acetate (I.1) and trans-(2-isobutyl-4-methyltetrahydropyran-4-yl) acetate (I.2), in which the weight fraction of cis-(2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight.

9. An isomerically pure trans-(2-isobutyl-4-methyltetrahydropyran-4-yl) acetate of the formula (I.2) or an isomer mixture of cis-(2-isobutyl-4-methyltetrahydropyran-4-yl) acetate (I.1) and trans-(2-isobutyl-4-methyltetrahydropyran-4-yl) acetate (I.2), in which the weight fraction of trans-(2-isobutyl-4-methyltetrahydropyran-4-yl) acetate (I.2), based on the total weight of cis-(2-isobutyl-4-methyltetrahydropyran-4-yl) acetate (I.1) and trans-(2-isobutyl-4-methyltetrahydropyran-4-yl) acetate (I.2), is in a range from 90% by weight to less than 100% by weight.

10. A fragrance or aroma substance composition, comprising
a) isomerically pure cis-(2-isobutyl-4-methyltetrahydropyran-4-yl) acetate of the formula (I.1) or isomerically pure trans-(2-isobutyl-4-methyltetrahydropyran-4-yl) acetate of the formula (I.2) or an isomer mixture of cis-(2-isobutyl-4-methyltetrahydropyran-4-yl) acetate (I.1) and trans-(2-isobutyl-4-methyltetrahydropyran-4-yl) acetate (1.2), where the weight fraction of cis-(2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight, or the weight fraction of trans-(2-isobutyl-4-methyltetrahydropyran-4-yl) acetate (I.2), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight,
b) optionally at least one further aroma chemical different from the compounds (I.1) and (I.2), and
c) optionally at least one diluent,
with the proviso that the composition comprises at least one component b) or c).

11. The composition according to claim 10, comprising the component a) in a weight fraction of from 0.1 to 70% by weight, preferably 1 to 50% by weight, based on the total weight of the composition.

12. A method for imparting and/or intensifying an odor or taste of a product with an iris-like note, a bergamot-like note, a linalyl acetate-like note, a woody note or a pepper/nutmeg-like note, in which the product is brought into contact with an organoleptically effective amount of an isomerically pure cis-(2-isobutyl-4-methyltetrahydropyran-4-yl) acetate of the formula (I.1) or an isomer mixture, where the weight fraction of cis-(2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2), is in a range from 90% by weight to less than 100% by weight.

13. A method for imparting and/or intensifying an odor or taste of a product with a jasmine-like note, a leather-like note or a strong fruit-like note, in which the product is brought into contact with an organoleptically effective amount of an isomerically pure trans-(2-isobutyl-4-methyltetrahydropyran-4-yl) acetate of the formula (I.2) or an isomer mixture, where the weight fraction of trans-(2-isobutyl-4-methyltetrahydropyran-4-yl) acetate (I.2), based on the total weight of cis-(2-isobutyl-4-methyltetrahydropyran-4-yl) acetate (I.1) and trans-(2-isobutyl-4-methyltetrahydropyran-4-yl) acetate (I.2) is in a range from 90% by weight to less than 100% by weight.

14. A perfumed or aromatized product, comprising an organoleptically effective amount of isomerically pure cis-(2-isobutyl-4-methyltetrahydropyran-4-yl) acetate of the formula (I.1) or of isomerically pure trans-(2-isobutyl-4-methyltetrahydropyran-4-yl) acetate of the formula (I.2) or of an isomer mixture of cis-(2-isobutyl-4-methyltetrahydropyran-4-yl) acetate (I.1) and trans-(2-isobutyl-4-methyltetrahydropyran-4-yl) acetate (I.2), as defined in any one of claims 1 to 6, or comprising an organoleptically effective amount of a fragrance or aroma substance composition as defined in either of claims 10 and 11.

15. The use of isomerically pure cis-(2-isobutyl-4-methyltetrahydropyran-4-yl) acetate of the formula (I.1) or of an isomer mixture, where the weight fraction of cis-(2-isobutyl-4-methyltetrahydropyran-4-yl) acetate (I.1), based on the total weight of cis-(2-isobutyl-4-methyltetrahydropyran-4-yl) acetate (I.1) and trans-(2-isobutyl-4-methyltetrahydropyran-4-yl) acetate (I.2), is in a range from 90% by weight to less than 100% by weight, for the partial or complete replacement of linalyl acetate in a fragrance or aroma substance composition comprising linalyl acetate or in a product aromatized or perfumed with linalyl acetate.

## Revendications

1. Utilisation d'acétate de cis-2-isobutyl-4-méthyl-tétrahydropyran-4-yle de formule (I.1) sous forme d'isomère pur ou d'acétate de trans-2-isobutyl-4-méthyl-tétrahydropyran-4-yle de formule (1.2) sous forme d'isomère pur ou d'un mélange d'isomères à base d'acétate de cis-2-isobutyl-4-méthyl-tétrahydropyran-4-yle (I.1) et d'acétate de trans-2-isobutyl-4-méthyl-tétrahydropyran-4-yle (I.2) 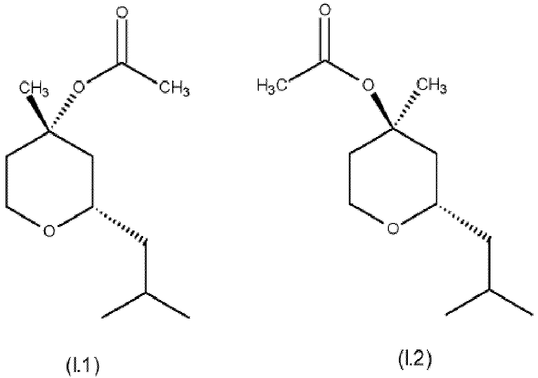
dans le cas de l'utilisation d'un mélange d'isomères la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situant dans une plage allant de 90 % en poids à moins de 100 % en poids, ou
la proportion en poids d'acétate de trans-2-isobutyl-4-méthyl-tétrahydropyran-4-yle (I.2) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situant dans une plage allant de 90 % en poids à moins de 100 % en poids,
en tant qu'arôme chimique.

2. Utilisation selon la revendication 1 dans des produits, choisis parmi des parfums, des produits de lavage et de nettoyage, des produits cosmétiques, des produits pour le soin du corps, des articles d'hygiène, des produits pour l'hygiène buccale et dentaire, des diffuseurs de parfum et des substances odorantes.

3. Utilisation selon la revendication 1 ou 2 d'acétate de cis-2-isobutyl-4-méthyl-tétrahydropyran-4-yle de formule (I.1) sous forme d'isomère pur ou d'un mélange d'isomères dans lequel la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids, pour la fabrication d'un parfum ayant une note florale, boisée ou citronnée.

4. Utilisation selon la revendication 1 ou 2 d'acétate de trans-2-isobutyl-4-méthyl-tétrahydropyran-4-yle de formule (I.2) sous forme d'isomère pur ou d'un mélange d'isomères dans lequel la proportion en poids d'acétate de trans-2-isobutyl-4-méthyl-tétrahydropyran-4-yle (I.2) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids, pour la fabrication d'un parfum ayant une note jasminée, une note cuir ou une puissante note fruitée.

5. Utilisation selon l'une quelconque des revendications précédentes, dans laquelle on utilise le composé de formule (I.1) sous forme de stéréoisomère pur ou sous forme de mélange d'isomères (2S,4S) et (2R,4R).

6. Utilisation selon l'une quelconque des revendications précédentes, dans laquelle on utilise le composé de formule (I.2) sous forme de stéréoisomère pur ou sous forme de mélange d'isomères (2S,4R) et (2R,4S).

7. Procédé pour la préparation d'acétate de cis-2-isobutyl-4-méthyl-tétrahydropyran-4-yle de formule (I.1) sous forme d'isomère pur ou d'acétate de trans-2-isobutyl-4-méthyl-tétrahydropyran-4-yle de formule (I.2) sous forme d'isomère pur ou d'un mélange d'isomères à base de (I.1) et (I.2), la proportion en poids de (I.1) ou la proportion en poids de (I.2) dans le mélange d'isomères, par rapport au poids total de (I.1) et (I.2), se situant dans une plage allant de 90 % en poids à moins de 100 % en poids,
dans lequel
i) on fait réagir un mélange d'isomères à base de cis-2-(2-méthylpropyl)-4-hydroxy-4-méthyl-tétrahydropyrane (II.1) et de trans-2-(2-méthyl-propyl)-4-hydroxy-4-méthyl-tétrahydropyrane (II.2) 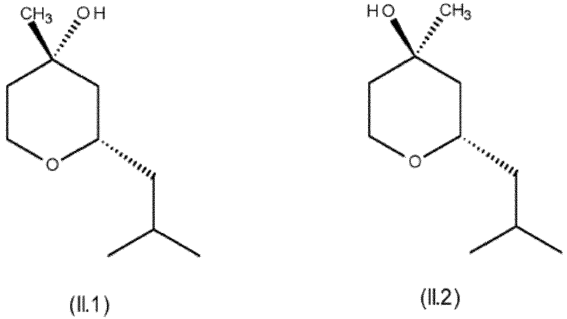 avec un composé de formule CH₃C(=O)-X, dans laquelle X représente Cl, Br ou CH₃C(=O)O,
ii) éventuellement on soumet à un fractionnement le mélange réactionnel obtenu dans l'étape i), pour obtenir au moins une fraction qui contient (I.1) sous forme d'isomère pur ou (I.2) sous forme d'isomère pur ou un mélange d'isomères à base de (I.1) et (I.2), la proportion en poids de (I.1) ou la proportion en poids de (I.2) dans le mélange d'isomères, par rapport au poids total de (I.1) et (I.2), se situant dans une plage allant de 90 % en poids à moins de 100 % en poids.

8. Acétate de cis-2-isobutyl-4-méthyl-tétrahydropyran-4-yle de formule (I.1) sous forme d'isomère pur ou mélanges d'isomères à base d'acétate de cis-2-isobutyl-4-méthyl-tétrahydropyran-4-yle (I.1) et d'acétate de trans-2-isobutyl-4-méthyl-tétrahydropyran-4-yle (I.2) dans lesquels la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids.

9. Acétate de trans-2-isobutyl-4-méthyl-tétrahydropyran-4-yle de formule (I.2) sous forme d'isomère pur ou mélanges d'isomères à base d'acétate de cis-2-isobutyl-4-méthyl-tétrahydropyran-4-yle (I.1) et d'acétate de trans-2-isobutyl-4-méthyl-tétrahydropyran-4-yle (I.2) dans lesquels la proportion en poids d'acétate de trans-2-isobutyl-4-méthyl-tétrahydropyran-4-yle (I.2) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids.

10. Composition de parfum ou de substance odorante, contenant
a) de l'acétate de cis-2-isobutyl-4-méthyl-tétrahydropyran-4-yle de formule (I.1) sous forme d'isomère pur ou de l'acétate de trans-2-isobutyl-4-méthyl-tétrahydropyran-4-yle de formule (I.2) sous forme d'isomère pur ou un mélange d'isomères à base d'acétate de cis-2-isobutyl-4-méthyl-tétrahydropyran-4-yle (I.1) et d'acétate de trans-2-isobutyl-4-méthyl-tétrahydropyran-4-yle (I.2) dans lequel la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids, ou la proportion en poids d'acétate de trans-2-isobutyl-4-méthyl-tétrahydropyran-4-yle (I.2) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids,
b) éventuellement un autre arôme chimique, différent des composés (I.1) et (I.2), et
c) éventuellement au moins un diluant,
étant entendu que la composition contient au moins un composant b) ou c).

11. Composition selon la revendication 10, contenant le composant a) en une proportion en poids de 0,1 à 70 % en poids, de préférence 1 à 50 % en poids, par rapport au poids total de la composition.

12. Procédé pour l'apport et/ou l'accentuation d'une odeur ou saveur d'un produit avec une note irisée, une note bergamote, une note d'acétate de linalyle, une note boisée ou une note poivrée/muscadée, dans lequel on met en contact le produit avec une quantité organoleptiquement efficace d'un acétate de cis-2-isobutyl-4-méthyl-tétrahydropyran-4-yle de formule (I.1) sous forme d'isomère pur ou d'un mélange d'isomères dans lequel la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids.

13. Procédé pour l'apport et/ou l'accentuation d'une odeur ou saveur d'un produit avec une note jasminée, une note cuir ou une puissante note fruitée, dans lequel on met en contact le produit avec une quantité organoleptiquement efficace d'un acétate de trans-2-isobutyl-4-méthyl-tétrahydropyran-4-yle de formule (I.2) sous forme d'isomère pur ou d'un mélange d'isomères dans lequel la proportion en poids d'acétate de trans-2-isobutyl-4-méthyl-tétrahydropyran-4-yle (I.2) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids.

14. Produit parfumé ou aromatisé, comprenant une quantité organoleptiquement efficace d'acétate de cis-2-isobutyl-4-méthyl-tétrahydropyran-4-yle de formule (I.1) sous forme d'isomère pur ou d'acétate de trans-2-isobutyl-4-méthyl-tétrahydropyran-4-yle (I.2) sous forme d'isomère pur ou d'un mélange d'isomères à base d'acétate de cis-2-isobutyl-4-méthyl-tétrahydropyran-4-yle (I.1) et d'acétate de trans-2-isobutyl-4-méthyl-tétrahydropyran-4-yle (I.2), tels que définis dans l'une quelconque des revendications 1 à 6, ou comprenant une quantité organoleptiquement efficace d'une composition de parfum ou d'arôme telle que définie dans l'une quelconque des revendications 10 et 11.

15. Utilisation d'acétate de cis-2-isobutyl-4-méthyl-tétrahydropyran-4-yle de formule (I.1) sous forme d'isomère pur ou d'un mélange d'isomères dans lequel la proportion en poids d'acétate de cis-2-isobutyl-4-méthyl-tétrahydropyran-4-yle (I.1) par rapport au poids total d'acétate de cis-2-isobutyl-4-méthyl-tétrahydropyran-4-yle (I.1) et d'acétate de trans-2-isobutyl-4-méthyl-tétrahydropyran-4-yle (I.2) se situe dans une plage allant de 90 % en poids à moins de 100 % en poids, pour le remplacement partiel ou total d'acétate de linalyle dans une composition de parfum ou d'arôme contenant de l'acétate de linalyle ou dans un produit parfumé ou aromatisé avec de l'acétate de linalyle.
